# EUROPÄISCHE PATENTSCHRIFT

(11) **EP 1 530 559 B1**
(45) Veröffentlichungstag und Bekanntmachung des Hinweises auf die Patenterteilung: **21.11.2007**
(21) Anmeldenummer: 03790832.4
(22) Anmeldetag: 30.07.2003
(51) Int. Cl.: C07C 51/09, C07C 53/02, C07C 53/06

(54) **VERFAHREN UND VORRICHTUNG ZUR HERSTELLUNG VON AMEISENSAUREN FORMIATEN UND DEREN VERWENDUNG**
METHOD AND DEVICE FOR PRODUCING FORMIC ACID FORMATES AND USE OF SAID FORMATES
PROCEDE ET DISPOSITIF DE PRODUCTION DE FORMIATES D'ACIDE FORMIQUE ET UTILISATION DESDITS FORMIATES

(30) Priorität: 12.08.2002 DE 10237379
(43) Veröffentlichungstag der Anmeldung: 18.05.2005
(73) Patentinhaber: BASF Aktiengesellschaft, 67056 Ludwigshafen (DE)
(72) Erfinder: ADAMI, Christoph, 69469 Weinheim (DE); KARL, Jörn, 67063 Ludwigshafen (DE); HAUK, Alexander, 67071 Ludwigshafen (DE); BÖHLING, Ralf, 64347 Griesheim (DE); PASTRE, Jörg, 64625 Bensheim (DE); LENZ, Robert, 67126 Hochdorf-Assenheim (DE)
(86) Internationale Anmeldenummer: PCT/EP2003/008399
(87) Internationale Veröffentlichungsnummer: WO 2004/020382

(56) Entgegenhaltungen:
- DE-A- 2 407 157
- DE-A- 2 653 448
- US-A- 2 545 889
- US-A- 4 218 568

## Beschreibung

Die vorliegende Erfindung betrifft ein Verfahren und eine Vorrichtung zur Herstellung von ameisensauren Formiaten ausgehend von Ameisensäuremethylester, Wasser und einer basischen Verbindung.

Ferner betrifft die Erfindung die Verwendung der ameisensauren Formiate zur Konservierung und/oder Ansäuerung von pflanzlichen und/oder tierischen Stoffen, zur Behandlung von Bioabfällen sowie als Additiv in der Tierernährung und/oder als Wachstumsförderer für Tiere.

Ameisensaure Formiate besitzen eine antimikrobielle Wirkung und werden beispielsweise eingesetzt zur Konservierung sowie zur Ansäuerung von pflanzlichen und tierischen Stoffen, wie etwa von Gräsern, landwirtschaftlichen Produkten oder Fleisch, zur Behandlung von Bioabfällen oder als Additiv zur Tierernährung.

Ameisensaure Formiate und Herstellmethoden für diese sind seit langem bekannt z.B. DE-A-2407157. So ist in Gmelins Handbuch der anorganischen Chemie, 8. Auflage, Nummer 21, Seiten 816 bis 819, Verlag Chemie GmbH, Berlin 1928 sowie Nummer 22, Seiten 919 bis 921, Verlag Chemie GmbH, Berlin 1937 die Darstellung von Natriumdiformiat sowie von Kaliumdiformiat durch Lösen von Natriumformiat sowie von Kaliumformiat in Ameisensäure beschrieben. Durch Temperaturerniedrigung beziehungsweise durch Abdampfen überschüssiger Ameisensäure sind die kristallinen Diformiate zugänglich.

DE 424 017 lehrt die Herstellung von ameisensauren Natriumformiaten mit verschiedenem Säuregehalt durch Einbringen von Natriumformiat in wässrige Ameisensäure in entsprechendem Molverhältnis. Durch Abkühlung der Lösung können die entsprechenden Kristalle erhalten werden.

Nach J. Kendall et al., Journal of the American Chemical Society, Vol. 43, 1921, Seiten 1470 bis 1481 sind ameisensaure Kaliumformiate durch Lösen von Kaliumcarbonat in 90%-iger Ameisensäure unter Bildung von Kohlendioxid zugänglich. Die entsprechenden Feststoffe können durch Kristallisation erhalten werden.

US 4,261,755 beschreibt die Herstellung von ameisensauren Formiaten durch Reaktion eines Überschusses an Ameisensäure mit dem Hydroxid, Carbonat oder Bicarbonat des entsprechenden Kations.

WO 96/35657 lehrt die Herstellung von Produkten, welche Disalze der Ameisensäure enthalten, durch Vermischen von Kalium-, Natrium-, Cäsium- oder Ammonium-Formiat, Kalium-, Natrium- oder Cäsium-hydroxid, -carbonat oder -bicarbonat oder Ammoniak mit gegebenenfalls wässriger Ameisensäure, anschließender Kühlung des Reaktionsgemisches, Filtration der erhaltenen Aufschlämmung und Trocknung des erhaltenen Filterkuchens sowie Rückführung des Filtrats.

Nachteilig an den obengenannten Verfahren ist, dass pro Mol gebildetem Formiat durch die Umsetzung mit den basischen Verbindungen jeweils ein Mol Ameisensäure verbraucht wird. Bekanntlich ist nämlich gerade die Herstellung von konzentrierter, das heißt weitgehend wasserfreier Ameisensäure ein apparativ aufwändiger, kosten- und energieintensiver Prozess. Somit sind die obengenannten Verfahren, bezogen auf die gesamte Wertschöpfungskette, apparativ sehr aufwändig sowie kosten- und energieintensiv.

DE-Az. 102 10 730.0 lehrt die Herstellung ameisensaurer Formiate durch Umsetzung von Ameisensäuremethylester mit Wasser und einer basischen Verbindung, welche einen pKₐ-Wert der korrespondierenden Säure der entsprechenden Dissoziationsstufe von ≥3 aufweist, und die anschließende Abtrennung des gebildeten Methanols sowie optional die Einstellung des gewünschten Säuregehalts durch Zugabe von Ameisensäure.

DE-Az. 101 54 757.9 lehrt die Herstellung von Metallformiat-Ameisensäure-Mischungen durch Carbonylierung des entsprechenden Metallhydroxids zum Metallformiat in Gegenwart von Wasser und eines Katalysators, destillative Abtrennung des Wassers und des Katalysators und Zumischen von Ameisensäure zum Metallformiat zur Gewinnung der gewünschten Metallformiat-Ameisensäure-Mischung.

Es bestand daher die Aufgabe, ein Verfahren bereitzustellen, welches die obengenannten Nachteile nicht mehr besitzt, die Herstellung ameisensaurer Formiate in industriellem Maßstab in hoher Ausbeute und hoher Raum-Zeit-Ausbeute, bei gleichzeitig großer Flexibilität bezüglich der Zusammensetzung und unter Einsatz gut zugänglicher Rohstoffe ermöglicht und eine einfache Verfahrensgestaltung mit niedrigen Investitionskosten und niedrigem Energiebedarf erlaubt.

Demgemäß wurde ein Verfahren zur Herstellung von ameisensauren Formiaten gefunden, das dadurch gekennzeichnet ist, dass man
(a) Ameisensäuremethylester mit Wasser partiell hydrolysiert;
(b) aus dem in der Verfahrensstufe (a) erhaltenen Reaktionsgemisch Ameisensäuremethylester und Methanol unter Bildung eines Ameisensäure und Wasser enthaltenden Stroms destillativ abtrennt;
(c) den Ameisensäuremethylester und gegebenenfalls Methanol enthaltenden Strom aus der Verfahrensstufe (b) durch
   (i) Umsetzung mit einer basischen Verbindung mit einem pKₐ-Wert der korrespondierenden Säure der entsprechenden Dissoziationsstufe von ≥ 3, gemessen bei 25°C in wässriger Lösung, in Gegenwart von Wasser, und
   (ii) destillativer Abtrennung des Methanols
   in einen Formiat und Wasser enthaltenden Strom überführt; und
(d) den Ameisensäure und Wasser enthaltenden Strom aus der Verfahrensstufe (b) und den Formiat und Wasser enthaltenden Strom aus der Verfahrensstufe (c) unter Bildung eines, das ameisensaure Formiat und Wasser enthaltenden Gemischs zusammenbringt.

Als ameisensaure Formiate sind Verbindungen und Gemische zu verstehen, welche Formiat-Anionen (HCOO-), Kationen (M^{x+}) und Ameisensäure (HCOOH) enthalten. Sie können zusammen in Form eines Feststoffs oder einer Flüssigkeit vorliegen und gegebenenfalls noch weitere Komponenten, wie beispielsweise weitere Salze, Zusatzsstoffe oder Lösungsmittel wie etwa Wasser, enthalten. Im Allgemeinen können die ameisensaure Formiate wiedergegeben werden durch die allgemeine Formel

HCOO-M^{x+}_{1/x} * y HCOOH (I),

in der M für ein ein- oder mehrwertiges, anorganisches oder organisches Kation steht, x eine positive ganze Zahl ist und die Ladung des Kations angibt und y den molaren Anteil an Ameisensäure bezogen auf das Formiat-Anion wiedergibt. Der molare Anteil an Ameisensäure bezogen auf das Formiat-Anion y liegt im Allgemeinen bei 0,01 bis 100, bevorzugt bei 0,05 bis 20, besonders bevorzugt bei 0,5 bis 5 und insbesondere bei 0,9 bis 3,1.

Die Natur des anorganischen oder organischen Kations M^{x+} ist prinzipiell unerheblich, sofern dieses unter den Bedingungen, unter denen das ameisensaure Formiat gehandhabt werden soll, stabil ist. Darunter ist beispielsweise auch die Stabilität gegenüber den reduzierend wirkendem Formiat-Anion zu verstehen. Als mögliche anorganische Kationen seien die ein- und/oder mehrwertigen Metallkationen der Metalle aus der Gruppe 1 bis 14 des Periodensystems, wie beispielsweise Lithium (Li⁺), Natrium (Na⁺), Kalium (K⁺), Cäsium (Cs⁺) , Magnesium (Mg²⁺), Kalzium (Ca²⁺) , Strontium (Sr²⁺) und Barium (Ba²⁺) , bevorzugt Natrium (Na⁺) , Kalium (K⁺), Cäsium (Cs⁺) und Kalzium (Ca²⁺) genannt. Als mögliche organische Kationen seien unsubstituiertes Ammonium (NH₄⁺) und durch ein oder mehrere kohlenstoff-enthaltende Reste, welche gegebenenfalls auch miteinander verbunden sein können, substituiertes Ammonium, wie beispielsweise Methylammonium, Dimethylammonium, Trimethylammonium, Ethylammonium, Diethylammonium, Triethylammonium, Pyrollidinium, N-Methylpyrroldinium, Piperidinium, N-Methylpiperidinium oder Pyridinium genannt.

Unter einem Kohlenstoff enthaltenden organischen Rest ist ein unsubstituierter oder substituierter, aliphatischer, aromatischer oder araliphatischer Rest mit 1 bis 30 Kohlenstoffatomen zu verstehen. Dieser Rest kann ein oder mehrere Heteroatome, wie etwa Sauerstoff, Stickstoff, Schwefel oder Phosphor enthalten, beispielsweise -O-, -S-, -NR-, -CO-, -N=, -PR- und/oder -PR₂ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe (bei dem Rest R handelt es sich hierbei ebenfalls um einen Kohlenstoff enthaltenden organischen Rest). Bei dem Kohlenstoff enthaltenden organischen Rest kann es sich um einen einwertigen oder auch mehrwertigen, beispielsweise zwei- oder dreiwertigen Rest handeln.

### Im Folgenden sind die einzelnen Verfahrensstufen näher erläutert:

Verfahrensstufe (a)

In der Verfahrensstufe (a) wird Ameisensäuremethylester mit Wasser partiell zu Ameisensäure und Methanol hydrolysiert. Unter partiell ist zu verstehen, dass nur ein Teil des zugeführten Ameisensäuremethylesters hydrolysiert wird.

Beim erfindungsgemäßen Verfahren können in der Verfahrensstufe (a) an sich die bekannten Verfahren zur Hydrolyse von Ameisensäuremethylester eingesetzt werden. Eine allgemeine Übersicht über bekannte und technisch relevante Verfahren zur Hydrolyse ist beispielsweise in Ullmann's Encyclopedia of Industrial Chemistry, 6^{th} edition, 2000 electronic release, Chapter "FORMIC ACID, Production" gegeben. Weitere geeignete Hydrolyseverfahren sind beispielsweise auch in EP-A 0 005 998 und EP-A 0 017 866 beschrieben.

Die Hydrolyse wird im Allgemeinen bei einer Temperatur von 80 bis 150°C und einem Druck von 0,5 bis 2,0 MPa abs durchgeführt. Als Reaktionsapparate können prinzipiell alle Reaktionsapparate eingesetzt werden, welche für Umsetzungen in der Flüssigphase geeignet sind. Als Beispiele seien Rührkessel und Strahlschlaufenreaktor genannt. Bevorzugt ist der Einsatz eines kaskadierten Reaktors.

Im Allgemeinen ist es vorteilhaft, die Hydrolyse in Gegenwart eines sauren Katalysators durchzuführen, da dieser die Hydrolysegeschwindigkeit signifikant erhöht. Als saure Katalysatoren können dabei die gebildete Ameisensäure oder zusätzliche Katalysatoren eingesetzt werden. Die zusätzlichen Katalysatoren können homogener oder heterogener Natur sein. Als Beispiele heterogener Katalysatoren seien saure Ionenaustauscher, wie etwa Polysulfonsäuren oder Poly(perfluoralkylen)sulfonsäuren (z.B. Nafion^{®} von Du Pont) und als Beispiele homogener Katalysatoren starke anorganische oder organische Säuren, wie etwa Schwefelsäure, Chlorwasserstoffsäure oder Alkyl- und Tolylsulfonsäuren genannt. Werden homogene Katalysatoren eingesetzt, so sind diese im Allgemeinen in einer Folgestufe abzutrennen. Je nach gewünschter Reinheit der herzustellenden ameisensauren Formiate ist es aber gegebenenfalls auch möglich, diese im System zu belassen. In diesem Fall finden sich die sauren Katalysatoren üblicherweise in Form ihrer Salze im ameisensauren Formiat wieder. Besonders bevorzugt wird die partielle Hydrolyse in Gegenwart von Ameisensäure als sauren Katalysator durchgeführt, wodurch die Zugabe eines zusätzlichen Katalysators und dessen anschließende Abtrennung beziehungsweise die eventuelle Verunreinigung der ameisensauren Formiate entfällt. Im Allgemeinen stellt man hierzu am Reaktoreingang eine Ameisensäure-Konzentration von etwa 0,1 bis 2 Gew.-%, bezogen auf das vorliegende, flüssige, Wasser und Ameisensäuremethylester enthaltende Gemisch, durch eine gezielte Zugabe von Ameisensäure beziehungsweise eines Ameisensäure enthaltenden Stroms ein.

Das beim erfindungsgemäßen Verfahren bei der Hydrolyse einzusetzende Molverhältnis von Ameisensäuremethylester zu Wasser beträgt im Allgemeinen 0,1 bis 10. Da es sich um eine Gleichgewichtsreaktion handelt, setzt man bevorzugt einen Überschuss an Wasser ein, wie beispielsweise auch aus der Lehre von EP-A 0 017 866 hervorgeht. Bevorzugt führt man in der Verfahrensstufe (a) den Ameisensäuremethylester und das Wasser in einem Molverhältnis von 0,1 bis 1 und besonders bevorzugt von 0,15 bis 0,3 zu.

Das aus der partiellen Hydrolyse erhaltene Reaktionsgemisch enthält somit nicht-umgesetzten Ameisensäuremethylester, Ameisensäure, Methanol sowie aufgrund des bevorzugten Einsatzes eines Wasser-Überschusses Wasser. Bevorzugt enthält das wässrige Reaktionsgemisch 5 bis 15 Mol-%, besonders bevorzugt 8 bis 12 Mol-% Ameisensäure, 3 bis 30 Mol-%, besonders bevorzugt 6 bis 12 Mol-% Ameisensäuremethylester und 6 bis 15 Mol-%, besonders bevorzugt 8 bis 12 Mol-% Methanol.

### Verfahrensstufe (b)

In der Verfahrensstufe (b) wird aus dem in der Verfahrensstufe (a) erhaltenen Reaktionsgemisch Ameisensäuremethylester und Methanol unter Bildung eines Ameisensäure und Wasser enthaltenden Stroms destillativ abgetrennt. Ameisensäuremethylester und Methanol können dabei prinzipiell zusammen in Form eines Stroms oder getrennt in Form eines Ameisensäuremethylester enthaltenden Stroms und eines Methanol enthaltenden Stroms abgetrennt werden. Im Allgemeinen werden Ameisensäuremethylester und Methanol im oberen Teil der Kolonne getrennt oder zusammen entnommen. Der Ameisensäure und Wasser enthaltende Strom wird im Allgemeinen aus dem Sumpf entnommen. Bevorzugt ist in der Verfahrensstufe (b) die gemeinsame Abtrennung eines Ameisensäuremethylester und Methanol enthaltenden Stroms.

Die Auslegung und der Betrieb der Destillationskolonne ist in erster Linie abhängig von der Zusammensetzung des zugeführten Stroms sowie den gewünschten Reinheiten der beiden Produktströme und kann vom Fachmann in bekannter Art und Weise ermittelt werden.

### Verfahrensstufe (c)

In der Verfahrensstufe (c) wird der Ameisensäuremethylester und gegebenenfalls Methanol enthaltende Strom aus der Verfahrensstufe (b) durch
(i) Umsetzung mit einer basischen Verbindung mit einem pKₐ-Wert der korrespondierenden Säure der entsprechenden Dissoziationsstufe von ≥3, gemessen bei 25°C in wässriger Lösung, in Gegenwart von Wasser, und
(ii) destillativer Abtrennung des Methanols
in einen Formiat und Wasser enthaltenden Strom überführt.

Die einzusetzende basische Verbindung weist bevorzugt einen pKₐ-Wert der korrespondierenden Säure der entsprechenden Dissoziationsstufe von ≥ 3,5, besonders bevorzugt von ≥ 9 und ganz besonders bevorzugt von ≥ 10, gemessen bei 25°C in wässriger Lösung, auf. Die basische Verbindung kann anorganischer oder organischer Natur sein. Bei der basischen Verbindung kann es sich um ein Salz oder eine kovalente Verbindung handeln. Unter der korrespondierenden Säure der entsprechenden Dissoziationsstufe ist dabei die durch formale Addition eines Protons (H⁺) gebildete Säure zu verstehen.

Für den Fall, dass es sich bei der basischen Verbindung um ein Salz handelt, kann dieses allgemein durch die Formel

M^{x+}ₐA^{a-}ₓ (II) ,

in der M und x die unter (I) genannte Bedeutung besitzen und A einem anorganischem oder organischem Anion mit der Ladung "a-" entspricht, dargestellt werden. Die korrespondierende Säure der entsprechenden Dissoziationsstufe entspricht somit HA^{(a-1)-}. Die entsprechende und für den heranzuziehenden pKₐ-Wert maßgebliche Dissoziationsgleichung lautet

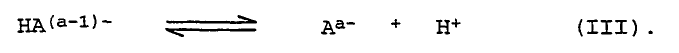

Für den Fall, dass es sich bei der basischen Verbindung um eine kovalente Verbindung B handelt, lautet die für den heranzuziehenden pKₐ-Wert maßgebliche Dissoziationsgleichung

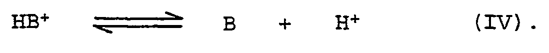

Als Beispiele geeigneter basischer Verbindungen seien die Salze M^{x+}ₐA^{a-}ₓ (II), in denen M^{x+} für ein ein- oder mehrwertiges Metallkation eines Metalls wie oben beschrieben und A^{a-} für ein Anion wie in Tabelle 1a aufgelistet steht sowie die kovalenten Verbindungen B wie in Tabelle 1b aufgelistet, genannt.

**Tabelle 1a: Mögliche Anionen A^{a-} geeigneter basischer Verbindungen und pKₐ-Werte (gemessen bei 25°C in wässriger Lösung) der korrespondierenden Säuren der entsprechenden Dissoziationsstufen.**

| Anionen A^{a-} | korrespondierende Säure | pKₐ-Wert |
|---|---|---|
| Hydroxid (OH⁻) | Wasser (H₂O) | 14,0 |
| Carbonat (CO₃²⁻) | Hydrogencarbonat (HCO₃⁻) | 10,3 |
| Hydrogencarbonat (HCO₃⁻) | Kohlensäure (H₂CO₃) | 6,4 |
| Borat (BO₃³⁻) | Hydrogenborat (HBO₃²⁻) | > 14 |
| Hydrogenborat (HBO₃²⁻) | Dihydrogenborat (H₂BO₃⁻) | > 14 |
| Dihydrogenborat (H₂BO₃⁻) | Borsäure (H₃BO₃) | 9,3 |
| Phosphat (PO₄³⁻) | Hydrogenphosphat (HPO₄²⁻) | 12,3 |
| Hydrogenphosphat (HPO₄²⁻) | Dihydrogenphosphat (H₂PO₄⁻) | 7,2 |
| Formiat | Ameisensäure | 3,8 |
| Acetat | Essigsäure | 4,8 |
| Propionat | Propionsäure | 4,9 |
| Oxalat (C₂O₄²⁻) | Hydrogenoxalat (HC₂O₄⁻) | 4,2 |
| 2-Ethylhexanoat (C₄H₉-CH(C₂H₅) -COO⁻) | 2-Ethylhexansäure (C₄H₉-CH(C₂H₅)-COOH) | > 4 |

**Tabelle 1b: Mögliche kovalente Basen B als geeignete basische Verbindungen und pKₐ-Werte (gemessen bei 25°C in wässriger Lösung) der korrespondierenden Säuren der entsprechenden Dissoziationsstufen.**

| kovalente Base B | korrespondierende Säure | pKₐ-Wert |
|---|---|---|
| Ammoniak | Ammonium | 9,3 |
| Methylamin | Methylammonium | 10,6 |
| Dimethylamin | Dimethylammonium | 10,7 |
| Trimethylamin | Trimethylammonium | 9,8 |
| Ethylamin | Ethylammonium | 10,7 |
| Diethylamin | Diethylammonium | 11,0 |
| Triethylamin | Triethylammonium | 10,8 |
| Pyrollidin | Pyrollidinium | 11,3 |
| N-Methylpyrroldin | N-Methylpyrroldinium | 10,3 |
| Piperidin | Piperidinium | 11,1 |
| N-Methylpiperidin | N-Methylpiperidinium | 10,1 |
| Pyridin | Pyridinium | 5,3 |

Bevorzugt setzt man beim erfindungsgemäßen Verfahren als basische Verbindungen Lithiumhydroxid, Lithiumhydrogencarbonat, Lithiumcarbonat, Natriumhydroxid, Natriumhydrogencarbonat, Natriumcarbonat, Kaliumhydroxid, Kaliumhydrogencarbonat, Kaliumcarbonat, Ammoniumcarbonat, Ammoniumhydrogencarbonat und/oder Ammoniak, besonders bevorzugt Natriumhydroxid, Natriumhydrogencarbonat, Natriumcarbonat, Kaliumhydroxid, Kaliumhydrogencarbonat, Kaliumcarbonat und/oder Ammoniak und besonders bevorzugt Natriumhydroxid, Natriumcarbonat, Kaliumhydroxid und/oder Kaliumcarbonat, insbesondere Natriumhydroxid und/oder Kaliumhydroxid ein.

Die Art der Zugabe der basischen Verbindungen ist beim erfindungsgemäßen Verfahren im Allgemeinen unwesentlich. Sie können in fester, flüssiger oder gasförmiger Form, als Reinsubstanz, als Substanzgemisch oder als Lösung zugegeben werden. Als Beispiele seien die Zugabe in Form wässriger Lösungen (z.B. wässrige Lösungen der Alkalisalze oder Ammoniakwasser), in Form fester Verbindungen (z.B. Pulver der Alkalisalze), in gasförmigem Zustand (z.B. gasförmiger Ammoniak) genannt. Bevorzugt ist die Zugabe in Form ihrer wässrigen Lösungen.

Auch die Reihenfolge der Zugaben der Edukte ist beim erfindungsgemäßen Verfahren im Allgemeinen unwesentlich. So ist es beispielsweise möglich, die basische Verbindung in fester oder flüssiger Form (z.B. als wässrige Lösung) vorzulegen und anschließend den Ameisensäuremethylester enthaltenden Strom flüssig oder gasförmig einzutragen. Es ist ferner möglich, den Ameisensäuremethylester enthaltenden Strom in flüssiger Form vorzulegen und anschließend die basische Verbindung zuzufügen. Des Weiteren ist es natürlich auch möglich und insbesondere bei der Durchführung eines kontinuierlich arbeitenden Verfahrens von Vorteil, den Ameisensäuremethylester enthaltenden Strom und die basische Verbindung kontinuierlich zusammenzuführen.

Das Molverhältnis des Ameisensäuremethylesters zur basischen Verbindung ist beim erfindungsgemäßen Verfahren vorteilhafterweise stöchiometrisch einzustellen, das heißt derart, dass sich entsprechend der Reaktionsstöchiometrie der zugegebene Ameisensäuremethylester mit der zugegebenen basischen Verbindung zum entsprechenden Formiat und Wasser umsetzt. Die maßgebliche Größe hierfür ist das sogenannte Moläquivalent der basischen Verbindung, wobei hierbei alle Dissoziationsstufen, welche durch Addition von Protonen zu korrespondierenden Säuren führen, welche einem pKₐ-Wert von ≥3, gemessen bei 25°C in wässriger Lösung, aufweisen, zu berücksichtigen sind. So ist beim Einsatz von Kaliumhydroxid als basische Verbindung bevorzugt ein Ameisensäuremethylester/Kaliumhydroxid-Molverhältnis von 1,0 zu wählen, da dies der Bildung von Kaliumformiat entspricht:

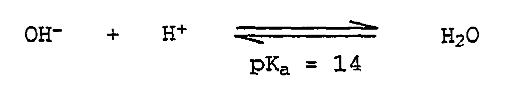

Beim Einsatz von Kaliumcarbonat als basische Verbindung ist bevorzugt ein Ameisensäuremethylester/Kaliumcarbonat-Molverhältnis von 2,0 zu wählen, da die korrespondierende Kohlensäure zweibasig ist:

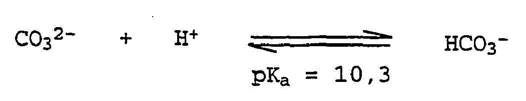

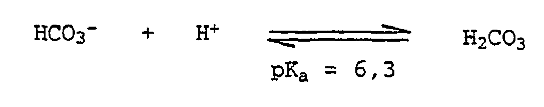

Abweichungen von der genannten stöchiometrischen Zugabe nach oben und unten sind beim erfindungsgemäßen Verfahren jedoch auch möglich. So besteht bei einem Unterschuss an basischer Verbindung die Gefahr einer unvollständigen Umsetzung des Ameisensäuremethylesters und somit die Gefahr einer Verunreinigung des destillativ abzutrennenden Methanols mit nicht-umgesetztem Ameisensäuremethylester. Bei einem Überschuss an basischer Verbindung würde der resultierende Strom neben dem entsprechenden Formiat und dem Wasser noch die restliche basische Verbindung enthalten.

Die Menge des beim erfindungsgemäßen Verfahren in der Verfahrensstufe (c) einzusetzenden Wassers kann über einen breiten Bereich variieren. Im Allgemeinen setzt man bei der Umsetzung 20 bis 90 Gew.-% und bevorzugt 40 bis 50 Gew.-% Wasser, bezogen auf die zugeführte Menge an Ameisensäuremethylester ein. Im Allgemeinen erfolgt die Zugabe des Wassers über eine wässrige Lösung der basischen Verbindung, obgleich auch die Zugabe von reinem Wasser möglich ist.

Die Umsetzung des Ameisensäuremethylester enthaltenden Stroms in Verfahrensstufe (c) mit der genannten basischen Verbindung in Gegenwart von Wasser führt man im Allgemeinen bei einer Temperatur von 0 bis 150°C, bevorzugt von 30 bis-120°C und besonders bevorzugt von 50 bis 80°C durch. Bei der Durchführung beträgt der Druck im Allgemeinen 0,05 bis 1 MPa abs, bevorzugt 0,08 bis 0,5 MPa abs und besonders bevorzugt 0,09 bis 0,15 MPa abs.

Die Umsetzung des Ameisensäuremethylester enthaltenden Stroms in Verfahrensstufe (c) mit der genannten basischen Verbindung in Gegenwart von Wasser ist prinzipiell unabhängig von der destillativen Abtrennung des Methanols. Die destillative Abtrennung des Methanols kann daher beim erfindungsgemäßen Verfahren prinzipiell vor, zusammen mit oder nach der genannten Umsetzung erfolgen. Bevorzugt ist die destillative Abtrennung des Methanols zusammen mit oder nach der genannten Umsetzung.

Bei der destillativen Abtrennung des Methanols vor oder nach der genannten Umsetzung können für die Umsetzung prinzipiell alle Reaktionsapparate eingesetzt werden, welche für Umsetzungen in der Flüssigphase geeignet sind. Als Beispiele genannt seien Rührkessel und Strahlschlaufenreaktor. Die destillative Abtrennung des Methanols erfolgt dabei dann in einem separaten Schritt, üblicherweise in einer Destillationskolonne.

Beim erfindungsgemäßen Verfahren besonders bevorzugt ist die Durchführung der destillativen Abtrennung des Methanols zusammen mit der Umsetzung des Ameisensäuremethylesters mit dem Wasser und der basischen Verbindung unter Überführung in den Formiat und Wasser enthaltenden Strom in einer Kolonne. Aufgrund des gegenüber Wasser niedrigeren Siedepunkts des Ameisensäuremethylesters gibt man hierbei den Ameisensäuremethylester und Methanol enthaltenden Strom aus der Verfahrensstufe (b) vorteilhafterweise unterhalb der Zugabestelle des Wassers und der basischen Verbindung zu. Da der Ameisensäuremethylester und das Methanol in der Kolonne aufsteigt und das Wasser sowie die basische Verbindung nach unten strömen, weist die Kolonne einen, für die genannte Umsetzung geeigneten Bereich auf. Das Methanol steigt nach oben und kann über Kopf isoliert werden. Da die Herstellung von Ameisensäuremethylester im Allgemeinen durch Carbonylierung von Methanol erfolgt, ist es besonders vorteilhaft, das über Kopf isolierte Methanol als Einsatzstoff für die Ameisensäuremethylester-Herstellung zurückzuführen, wobei das zurückzuführende Methanol bei dieser Variante durchaus auch noch restliche Mengen an Ameisensäuremethylester enthalten kann. Somit ist es in der Gesamtbilanz lediglich erforderlich, die geringen Methanol-Verluste durch frisches Methanol zu ersetzen.

Der das wässrige Formiat enthaltende Strom strömt in der Kolonne nach unten und wird als Sumpfstrom entnommen. Dabei ist es gegebenenfalls von Vorteil, einen Teil des Wassers als Seitenstrom am unteren Ende der Kolonne zu entnehmen und der Hydrolyse zurückzuführen. Durch diese Maßnahme kann bereits eine höher konzentrierte wässrige Lösung des entsprechenden Formiats erhalten werden.

Die erforderliche Verweilzeit im Verseifungsteil der Kolonne kann beispielsweise durch geeignete Einbauten, wie etwa Thormann-Böden, oder gegebenenfalls durch ein externes Reaktionsvolumen bereitgestellt werden. Bei Bereitstellung eines externen Reaktionsvolumens wird der zu verseifende Strom aus der Kolonne an geeigneter Stelle durch eine Seitenabzug entnommen, dem externen Reaktionsapparat zugeführt und der Kolonne an geeigneter Stelle wieder zugeführt. Im Rahmen der vorliegenden Erfindung seien beide Varianten als in erster Linie gleichwertig anzusehen.

Die Auslegung der Kolonne erfolgt in der für den Fachmann üblichen und bekannten Art und Weise.

### Verfahrensstufe (d)

In der Verfahrensstufe (d) wird der die Ameisensäure und das Wasser enthaltende Strom aus der Verfahrensstufe (b) und der das Formiat und Wasser enthaltende Strom aus der Verfahrensstufe (c) unter Bildung eines, das ameisensaure Formiat und Wasser enthaltenden Gemischs zusammengebracht.

Die Reihenfolge der Zugaben des Ameisensäure und das Wasser enthaltenden Stroms aus der Verfahrensstufe (b) und des, das Formiat und Wasser enthaltenden Stroms aus der Verfahrensstufe (c) ist beim erfindungsgemäßen Verfahren im Allgemeinen unwesentlich. Insbesondere ist es möglich und gegebenenfalls vorteilhaft, den die Ameisensäure und das Wasser enthaltenden Strom aus der Verfahrensstufe (b) und/oder den das Formiat und Wasser enthaltenden Strom aus der Verfahrensstufe (c) vor dem Zusammenbringen einer Aufkonzentration an Ameisensäure beziehungsweise Formiat zu unterziehen. Hierzu sei insbesondere die Entfernung eines Teils des vorhandenen Wassers durch Verdampfung, bevorzugt durch Abdestillation, genannt.

Temperatur und Druck sind für das Zusammenbringen in der Verfahrensstufe (d) im Allgemeinen unwesentlich. Im Allgemeinen erfolgt das Zusammenbringen bei einer Temperatur von 0 bis 150°C und einem Druck von 0,01 bis 0,3 MPa abs.

Als Apparate können prinzipiell alle Apparate eingesetzt werden, welche für Umsetzungen in der Flüssigphase sowie gegebenenfalls für Umsetzungen in der Flüssigphase unter gleichzeitiger Abtrennung einer verdampfbaren Komponente geeignet sind. Als Beispiele seien Rührkessel, Strahlschlaufenreaktoren und Kolonnen genannt. Des Weiteren ist es beispielsweise auch möglich, die beiden Ströme durch Zusammenfluss innerhalb einer Rohrleitung, vorteilhafterweise mit nachgeschalteter Mischstrecke, zu vereinen. Ferner ist es auch möglich, die beiden Ströme in dem Apparat zusammenzuführen, in dem auch die Isolation von festem ameisensauren Formiat erfolgt.

Das durch Zusammenbringen des, die Ameisensäure und das Wasser enthaltenden Strom aus der Verfahrensstufe (b) und des, das Formiat und Wasser enthaltenden Strom aus der Verfahrensstufe (c) erhaltene Gemisch enthält das ameisensaure Formiat in Form einer wässrigen Lösung, gegebenenfalls mit bereits ausgefallenem ameisensauren Formiat als Feststoff. Je nach Bedarf kann es in dieser Form abgefüllt, gelagert, transportiert und/oder für entsprechende Formulierungen oder Anwendungen eingesetzt werden. Des Weiteren kann das ameisensaure Formiat durch nachgeschaltete Verfahrensschritte weiter aufkonzentriert beziehungsweise als Feststoff isoliert werden.

Bevorzugt ist eine Variante, bei der man in der Verfahrensstufe (d)
(i) den, die Ameisensäure und das Wasser enthaltenden Strom aus der Verfahrensstufe (b) zusammen mit der aus Schritt (iv) zurückgeführten Mutterlauge in einer Kolonne oder einem Verdampfer unter destillativer Abtrennung von Wasser aufkonzentriert;
(ii) den aus Schritt (i) durch Aufkonzentration gewonnen, Ameisensäure, Wasser und Formiat enthaltenden Strom mit dem, das Formiat und Wasser enthaltenden Strom aus der Verfahrensstufe (c) unter Bildung eines, das ameisensaure Formiat und Wasser enthaltende Gemisch zusammenbringt;
(iii) festes ameisensaures Formiat aus dem aus Schritt (ii) erhaltenen ameisensauren Formiat und Wasser enthaltenden Gemisch durch Kristallisation abscheidet und dieses isoliert; und
(iv) die erhaltene Mutterlauge zu Schritt (i) zurückführt.

Die Kolonne beziehungsweise der Verdampfer in Schritt (i) ist im Allgemeinen derart zu betreiben ist, dass ein Teil des zugeführten Wassers, beispielsweise über Kopf, abgezogen werden kann. Der verbleibende, Ameisensäure, Wasser und Formiat enthaltende Strom weist im Allgemeinen einen Wassergehalt von 10 bis 40 Gew.-% auf und wird als Sumpfprodukt entnommen. Die genannte Fahrweise besitzt den Vorteil einer gewissen Aufkonzentrierung des die Ameisensäure und das Formiat enthaltenden Stroms. Das aus der Kolonne oder dem Verdampfer entnommene Wasser wird vorteilhafterweise der Hydrolysestufe in Verfahrensschritt (a) zurückgeführt und der Überschuss aus dem Verfahren abgezogen. Die Auslegung der Kolonne beziehungsweise des Verdampfers erfolgt in der für den Fachmann üblichen und bekannten Art und Weise.

Das Zusammenbringen des, durch Aufkonzentration gewonnen, Ameisensäure, Wasser und Formiat enthaltenden Stroms mit dem, das Formiat und Wasser enthaltenden Strom aus der Verfahrensstufe (c) unter Bildung eines, das ameisensaure Formiat und Wasser enthaltende Gemischs in Schritt (ii) kann beispielsweise zwischen der Kolonne und dem Kristallisationsapparat, beispielsweise durch Zusammenführung zweier Leitungen oder in einem separaten Mischapparat, oder im Kristallisationsapparat selbst erfolgen.

Die Durchführung der Kristallisation ist dem Fachmann allgemein bekannt, wobei die genaue Auslegung und Fahrweise in der üblichen Art und Weise erfolgen kann. Im Allgemeinen führt man die Kristallisation bei einer Temperatur im Bereich von -20°C bis +80°C und bevorzugt von 0°C bis 60°C durch. In der Regel nimmt die Menge an auskristallisiertem Produkt mit fallender Temperatur zu. Die Kristallisation kann prinzipiell in allen bekannten Apparaten hierzu durchgeführt werden. Die genannte Ausführungsform ist besonders vorteilhaft einsetzbar zur Abtrennung von ameisensauren Formiaten, welche in der gewünschten Zusammensetzung kristallisierbar sind. Als relevante Beispiele seien Kaliumdiformiat (HCOOK * HCOOH), Natriumdiformiat (HCOONa * HCOOH), Natriumtetraformiat (HCOONa * 3 HCOOH) oder deren Gemische genannt. Die Abtrennung der auskristallisierten Formiate oder ameisensauren Formiate geschieht im Allgemeinen durch die üblichen und bekannten Methoden, wie beispielsweise durch Filtration oder Zentrifugation.

Die Mutterlauge, welche bei der Kristallisation des festen ameisensauren Formiats anfällt, wird in Schritt (iv) zu Schritt (i) zurückgeführt. Da diese noch einen beträchtlichen Anteil an Wertprodukt enthält, wird somit auch dessen Isolierung sichergestellt. Alternativ ist jedoch auch möglich, das in der Mutterlauge befindliche Wertprodukt auf andere Art und Weise zu nutzen, beispielsweise durch direkte Nutzung als Lösung.

Ebenfalls bevorzugt ist eine Variante, bei der man in der Verfahrensstufe (d)
(i) den, die Ameisensäure und das Wasser enthaltenden Strom aus der Verfahrensstufe (b) und den, das Formiat und das Wasser enthaltenden Strom aus der Verfahrensstufe (c) zu einem, das ameisensaure Formiat und Wasser enthaltende Gemisch in einer Kolonne oder einem Verdampfer unter destillativer Abtrennung von Wasser zusammenbringt; und
(ii) festes ameisensaures Formiat aus dem aus Schritt (i) erhaltenen ameisensauren Formiat und Wasser enthaltenden Gemisch durch Sprühgranulation, Sprühtrocknung oder Schmelzkristallisation abscheidet und dieses isoliert.

Das Zusammenbringen der beiden Ströme in Schritt (i) kann vor der Kolonne beziehungsweise dem Verdampfer, beispielsweise durch Zusammenführung zweier Leitungen oder in einem separaten Mischapparat, oder in der Kolonne beziehungsweise dem Verdampfer, beispielsweise durch zwei getrennte Zuführungen, erfolgen.

Die Kolonne beziehungsweise der Verdampfer in Schritt (i) ist im Allgemeinen derart zu betreiben ist, dass ein Teil des zugeführten Wassers, beispielsweise über Kopf, abgezogen werden kann. Das verbleibende, ameisensaure Formiat enthaltende Gemisch, welches im Allgemeinen einen Wassergehalt von 0,5 bis 30 Gew.-% aufweist, wird als Sumpfprodukt entnommen. Insbesondere bei der Isolierung des ameisensauren Formiats mittels Schmelzkristallisation wird im Sumpfprodukt ein geringer Wassergehalt von im Allgemeinen ≤ 1 Gew.-% eingestellt. Die genannte Fahrweise besitzt den Vorteil einer gewissen Aufkonzentrierung des, das ameisensaure Formiat enthaltenden Stroms. Das aus der Kolonne oder dem Verdampfer entnommene Wasser wird vorteilhafterweise der Hydrolysestufe in Verfahrensschritt (a) zurückgeführt und der Überschuss aus dem Verfahren abgezogen. Die Auslegung der Kolonne beziehungsweise des Verdampfers erfolgt in der für den Fachmann üblichen und bekannten Art und Weise.

Die Durchführung der Sprühgranulation, Sprühtrocknung und Schmelzkristallisation ist dem Fachmann allgemein bekannt, wobei die genaue Auslegung und Fahrweise in der üblichen Art und Weise erfolgen kann. Auch die oben genannten Methoden sind besonders vorteilhaft einsetzbar zur Abtrennung von ameisensauren Formiaten, welche in der gewünschten Zusammensetzung kristallisierbar sind. Als relevante Beispiele seien Kaliumdiformiat (HCOOK * HCOOH), Natriumdiformiat (HCOONa * HCOOH), Natriumtetraformiat (HCOONa * 3 HCOOH) oder deren Gemische genannt.

Da bei der Sprühgranulation, der Sprühtrocknung sowie der Schmelzkristallisation vorteilhafterweise ein wässriges ameisensaures Formiat mit einem geringen Wassergehalt eingesetzt werden kann, wird im Allgemeinen auch nur ein geringer Anteil an Kondensat beziehungsweise freier Ameisensäure erhalten. Je nach der anfallenden Menge und der vorliegenden Restkonzentration an ameisensauren Formiat ist es gegebenenfalls auch vorteilhaft, den Strom nicht rückzuführen, sondern aus dem System auszuschleusen.

Das erfindungsgemäße Verfahren kann prinzipiell diskontinuierlich, halbkontinuierlich oder kontinuierlich durchgeführt werden. Bevorzugt führt man das erfindungsgemäße Verfahren kontinuierlich durch.

Bevorzugt stellt man beim erfindungsgemäßen Verfahren als ameisensaures Formiat ameisensaure Metallformiate, besonders bevorzugt ameisensaures Kaliumformiat, ameisensaures Natriumformiat, ameisensaures Kalziumformiat oder deren Gemische und ganz besonders bevorzugt Kaliumdiformiat (HCOOK * HCOOH), Natriumdiformiat (HCOONa * HCOOH), Natriumtetraformiat (HCOONa * 3 HCOOH) oder deren Gemische her.

Die ameisensauren Formiate werden im Allgemeinen in Form ihrer Lösungen oder kristallin als Feststoffe hergestellt. Sie können gegebenenfalls noch mit weiteren Komponenten, wie beispielsweise weiteren Formiatsalzen versetzt werden. Bei den kristallinen ameisensauren Formiaten ist es in der Regel für die Lagerung, den Transport und den Einsatz vorteilhaft, diese zusammen mit einem Trocknungsmittel, beispielsweise Silicate oder Stärke, zu einem partikulären Kompaktat oder diversen Formkörpern, wie etwa Tabletten oder Kugeln, zu verdichten.

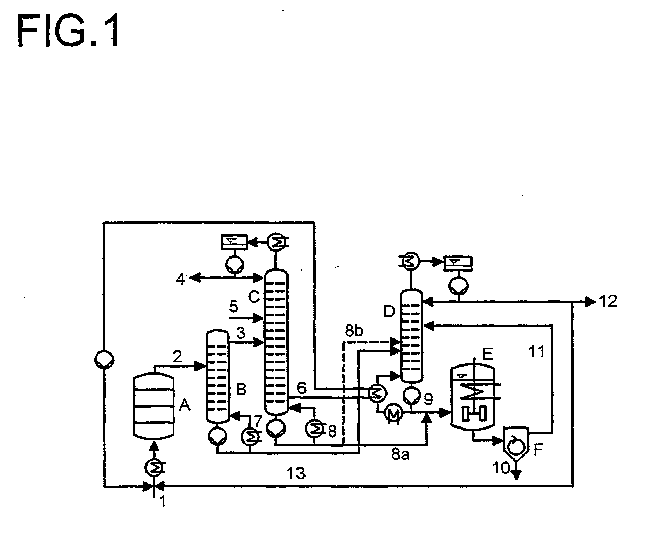

In einer besonders bevorzugten Ausführungsform, deren vereinfachtes Verfahrensfließbild in Abbildung 1 dargestellt ist, gibt man über Leitung (1) Ameisensäuremethylester sowie vom Verfahren rückgeführtes, Ameisensäure enthaltendes Wasser dem kaskadierten Hydrolysereaktor (A) zu. Im Allgemeinen werden die beiden Edukte vorgemischt (wie im Fließbild dargestellt) oder getrennt in einem Wärmetauscher auf die gewünschte Eintrittstemperatur gebracht. Das aus der Hydrolysestufe (Verfahrensstufe (a)) stammende Reaktionsgemisch, welches nicht-umgesetzten Ameisensäuremethylester, Wasser, Ameisensäure und Methanol enthält, wird über Leitung (2) der Kolonne (B) zugeführt, in der eine destillative Trennung des Reaktiongemischs in einen Ameisensäuremethylester und Methanol enthaltenden Kopfstrom und einen wässrige Ameisensäure enthaltenden Sumpfstrom erfolgt (Verfahrensstufe (b)). Der Ameisensäuremethylester und Methanol enthaltende Kopfstrom wird über Leitung (3) der Kolonne (C) zugeführt. Des Weiteren wird der Kolonne (C) oberhalb der Zulaufstelle des Ameisensäuremethylester und Methanol enthaltende Stroms über Leitung (5) die wässrige basische Verbindung, besonders bevorzugt Kaliumhydroxidlösung, zugeführt. Über Kopf von Kolonne (C) wird Methanol gewonnen und bevorzugt zur erneuten Herstellung von Ameisensäuremethylester durch Carbonylierung rückgeführt. Am unteren Ende der Kolonne (C) wird ein Teil des Wassers entnommen und über Leitung (6) zur Hydrolysestufe zurückgeführt. Als Sumpfprodukt wird eine wässrige Kaliumformiatlösung erhalten. Der die wässrige Ameisensäure enthaltende Strom aus der Verfahrensstufe (b) wird über Leitung (7) der Kolonne (D) zugeführt. Gegebenenfalls erfolgt über Leitung (8) und (8b) auch eine Zufuhr eines Teils des, die wässrige Formiatlösung enthaltenden Stroms aus der Verfahrensstufe (c). Die Kolonne (D) wird vorteilhafterweise derart betrieben, dass als Sumpfprodukt ein aufkonzentriertes, Ameisensäure, Formiat und Wasser enthaltendes Gemisch mit einen Wassergehalt von im Allgemeinen 10 bis 40 Gew.-% erhalten wird. Ein Teil des Wassers wird der Kolonne (D) in Form eines Ameisensäure enthaltenden Wasser-Stroms als Kopfprodukt entnommen und über Leitung (13) zur Hydrolysestufe zurückgeführt. Ein Teil des geringe Mengen an Ameisensäure enthaltenden Wasser-Stroms kann dabei optional über Leitung (12) aus dem System entnommen werden. Das Sumpfprodukt der Kolonne (D) wird über Leitung (9) einem zur Kristallisation geeigenetem Apparat (E), beispielsweise einem sogenannten Kühlscheiben-Kristaller, zugeführt. Über Leitung (8a) erfolgt die Zufuhr des, die wässrige Formiatlösung enthaltenden Stroms aus der Verfahrensstufe (c). Die Zufuhr kann dabei beispielsweise durch Zusammenführung zweier Leitungen (wie in Abbildung 1 dargestellt) oder direkt im Kristallisationsapparat erfolgen. Die Kristallisation erfolgt in erster Linie durch Temperaturabsenkung. Die erhaltenen Kristalle werden zusammen mit der überstehenden Lösung zur Abtrennung dem Apparat (F) zugeführt. Bevorzugt erfolgt die Abtrennung durch Zentrifugation. Die abgetrennten Kristalle werden über Leitung (10) entnommen und können beispielsweise in optionalen Folgestufen getrocknet und/oder konfektioniert werden. Die erhaltene Mutterlauge wird über Leitung (11) zur Kolonne (D) zurückgeführt.

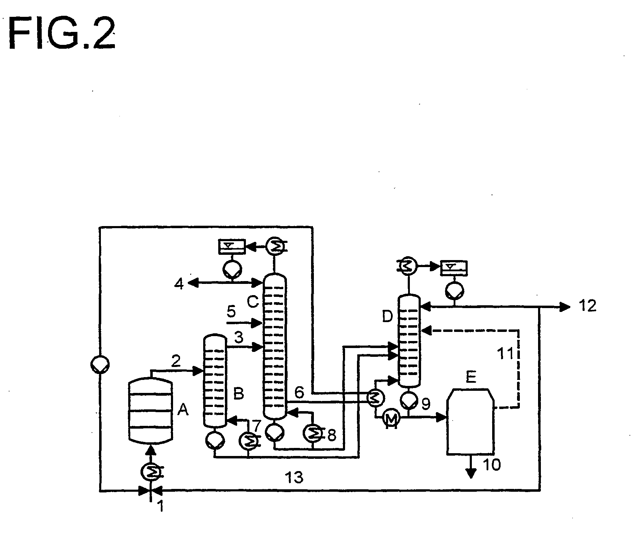

In einer anderen, besonders bevorzugten Ausführungsform, deren vereinfachtes Verfahrensfließbild in Abbildung 2 dargestellt ist, führt man die Verfahrensstufen (a), (b) und (c) wie in der zuvor beschriebenen, besonders bevorzugten Ausführungsform durch. Der, die wässrige Ameisensäure enthaltende Strom aus der Verfahrensstufe (b) wird über Leitung (7) und der, die wässrige Formiatlösung enthaltende Strom aus der Verfahrensstufe (c) über Leitung (8) der Kolonne (D) zugeführt. Die Kolonne (D) wird vorteilhafterweise derart betrieben, dass als Sumpfprodukt ein aufkonzentriertes, Ameisensäure, Formiat und Wasser enthaltendes Gemisch mit einen Wassergehalt von im Allgemeinen 0,5 bis 30 Gew.-% erhalten wird. Ein Teil des zugeführten Wassers wird der Kolonne (D) in Form eines Ameisensäure enthaltenden Wasser-Stroms als Kopfprodukt entnommen und über Leitung (13) zur Hydrolysestufe zurückgeführt. Ein Teil des geringe Mengen an Ameisensäure enthaltenden Wasser-Stroms kann dabei optional über Leitung (12) aus dem System entnommen werden. Das Sumpfprodukt der Kolonne (D) wird über Leitung (9) einem zur Sprühgranulation, Sprühtrocknung oder Schmelzkristallisation geeigenetem Apparat (E) zugeführt. Das erhaltene feste ameisensaure Formiat wird über Leitung (10) entnommen und kann beispielsweise in optionalen Folgestufen weiter getrocknet und/oder konfektioniert werden. Das erhaltene Kondensat kann optional über Leitung (11) zur Kolonne (D) zurückgeführt oder aus dem System ausgeschleust werden.

Das erfindungsgemäße Verfahren ermöglicht die Herstellung ameisensaurer Formiate in industriellem Maßstab in hoher Ausbeute und hoher Raum-Zeit-Ausbeute, bei gleichzeitig großer Flexibilität bezüglich der Zusammensetzung und unter Einsatz gut zugänglicher Rohstoffe bei einfacher Verfahrensgestaltung und niedrigen Investitionskosten. Das Verfahren besitzt des Weiteren den entscheidenden Vorteil, dass sowohl das Formiat als auch die Ameisensäure direkt aus dem Ameisensäuremethylester ohne den kostenintensiven und apparativ aufwändigen Umweg über die konzentrierte Ameisensäure gewonnen werden kann. Das erfindungsgemäße Verfahren ist daher verfahrenstechnisch einfach durchführbar und weist gegenüber den Verfahren unter direktem Einsatz von konzentrierter Ameisensäure nach dem Stand der Technik deutlich geringere Investitionskosten und einen deutlich niedrigeren Energiebedarf auf. Ferner kann teilweise auf den Einsatz hochlegierter Stähle verzichtet werden, da die ameisensauren Formiate weitaus weniger korrosiv sind als konzentrierte Ameisensäure.

Ferner ist Gegenstand der Erfindung eine Vorrichtung zur Herstellung der ameisensauren Formiate gemäß dem erfindungsgemäßen Verfahren, umfassend
(a) einen, zur Hydrolyse von Ameisensäuremethylester geeigneten Reaktor (A);
(b) eine, zur destillativen Trennung eines Ameisensäuremethylester, Ameisensäure, Methanol und Wasser enthaltenden Stroms in Ameisensäuremetyhlester, Methanol und einen Ameisensäure und Wasser enthaltenden Strom geeignete Kolonne (B), welche zulaufseitig mit dem Reaktor (A) verbunden ist;
(c) eine, zur Verseifung von Ameisensäuremethylester mit einer basischen Verbindung und zur destillativen Abtrennung von Methanol geeigneten Kolonne (C), welche zulaufseitig mit dem Kolonnenkopf der Kolonne (B) verbunden ist und oberhalb des genannten Zulaufs eine Zulauf stelle für die basische Verbindung aufweist; und
(d) eine, zur Abtrennung von Wasser aus einem Ameisensäure und Wasser enthaltenden Strom geeignete Kolonne (D), welche zulaufseitig mit dem Kolonnensumpf der Kolonne (B) verbunden ist.

Als geeigneter Reaktor (A) sei beispielsweise ein Rührkessel oder ein Strahlschlaufenreaktor genannt. Bevorzugt ist ein kaskadierter Reaktor. Die Auslegung des Reaktors (A) erfolgt nach der für den Fachmann üblichen und bekannten Art und Weise.

Die Auslegung der Kolonne (B) erfolgt nach der für den Fachmann üblichen und bekannten Art und Weise.

Die Kolonne (C) kann zur Bereitstellung der für das Verfahren erforderlichen Verweilzeit im Verseifungsteil geeignete Einbauten, wie etwa Thormann-Böden, oder gegebenenfalls ein, an die Kolonne angeschlossenes externes Reaktionsvolumen umfassen. Das gegebenenfalls vorhandene externe Reaktionsvolumen ist im Allgemeinen durch einen geeigneten Seitenabzug und einer geeigneten Seitenzufuhr mit der Kolonne verbunden. Die Auslegung der Kolonne (C) erfolgt nach der für den Fachmann üblichen und bekannten Art und Weise.

Die Auslegung der Kolonne (D) erfolgt nach der für den Fachmann üblichen und bekannten Art und Weise.

Als Vorrichtung bevorzugt ist eine Vorrichtung, welche zusätzlich zu den oben genannten Merkmalen (a) bis (d)
(e) einen, zur Kristallisation von ameisensauren Formiat geeigneten Apparat (E), welcher zulaufseitig mit dem Kolonnensumpf der Kolonne (D) und mit dem Kolonnensumpf der Kolonne (C) verbunden ist;
(f) einen, zur Abtrennung von Kristallen des ameisensauren Formiats geeigneten Apparat (F), welcher zulaufseitig mit Apparat (E) verbunden ist; und
(g) eine, zur Rückführung von Mutterlauge geeignete Verbindungsleitung (11) zwischen Apparat (F) und Kolonne (D)
umfasst.

Die Auslegung der Apparate (E) und (F) erfolgt nach der für den Fachmann üblichen und bekannten Art und Weise.

Des Weiteren ist als Vorrichtung bevorzugt eine Vorrichtung, welche zusätzlich zu den oben genannten Merkmalen (a) bis (d)
(e) eine, zur Zuführung von wässrigem Formiat geeignete Verbindungsleitung (8) zwischen dem Kolonnensumpf der Kolonne (C) und der Kolonne (D); und
(f) einen, zur Sprühgranulation, Sprühtrocknung oder Schmelzkristallisation geeigneten Apparat (E), welcher zulaufseitig mit derm Kolonnensumpf der Kolonne (D) verbunden ist
umfasst.

Die Auslegung des Apparats (E) erfolgt nach der für den Fachmann üblichen und bekannten Art und Weise.

Die erfindungsgemäß hergestellten ameisensauren Formiate können verwendet werden zur Konservierung und/oder Ansäuerung von pflanzlichen und tierischen Stoffen. Als Beispiele seien die Verwendung ameisensauren Formiate zur Konservierung und Ansäuerung von Gras, landwirtschaftlichen Pflanzen, Fisch sowie Fisch- und Fleischprodukten genannt, wie sie beispielsweise in WO 97/05783, WO 99/12435, WO 00/08929 und WO 01/19207 beschrieben sind.

Die erfindungsgemäß hergestellten ameisensauren Formiate können ferner verwendet werden zur Behandlung von Bioabfällen. Die Verwendung ameisensaurer Formiate zur Behandlung von Bioabfällen ist beispielsweise in WO 98/20911 beschrieben.

Die erfindungsgemäß hergestellten ameisensauren Formiate können des weiteren verwendet werden als Additiv in der Tierernährung und/oder als Wachstumsförderer für Tiere, wie beispielsweise für Zuchtsauen, Mastschweine, Geflügel, Kälber, Kühe und Fische. Die genannte Verwendung ist beispielsweise in WO 96/35337 beschrieben. Bevorzugt ist die Verwendung der erfindungsgemäß hergestellten ameisensauren Kaliumformiate, insbesondere von Kaliumdiformiat, als Additiv in der Tierernährung und/oder als Wachstumsförderer für Tiere, insbesondere für Zuchtsauen und Mastschweine.

Als ganz besonder bevorzugte Mischungen für die bevorzugte Verwendung der nach dem erfindungsgemäßen Verfahren hergestellten ameisensauren Kaliumformiate als Additiv in der Tierernährung

Als ganz besonder bevorzugte Mischungen für die bevorzugte Verwendung der nach dem erfindungsgemäßen Verfahren hergestellten ameisensauren Kaliumformiate als Additiv in der Tierernährung und/oder als Wachstumsförderer für Tiere seien die folgenden zwei Zusammensetzungen genannt:

| | Mischung 1 (Gew.-%) | Mischung 2 (Gew.-%) |
|---|---|---|
| Kaliumdiformiat | 20 bis 60 | 60 bis 99 |
| Natriumdiformia/tetraformiat | 20 bis 50 | --- |
| Kalziumformiat | 0 bis 25 | 0 bis 28 |
| Trocknungsmittel (Silicat oder Stärke) | 0 bis 4 | 0 bis 4 |
| Wasser | 0 bis 5 | 0 bis 5 |

Ganz besonders bevorzugt ist die Verwendung des erfindungsgemäß hergestellten Kaliumdiformiats als Additiv in der Tierernährung und/oder als Wachstumsförderer für Tiere in Form eines Produkts der Zusammensetzung 98,0 ± 1 Gew.-% Kaliumdiformiat, 1,5 ± 1 Gew.-% Silicat und 0,5 ±0,3 Gew.-% Wasser.

## Patentansprüche

1. Verfahren zur Herstellung von ameisensauren Formiaten, **dadurch gekennzeichnet, dass** man
(a) Ameisensäuremethylester mit Wasser partiell hydrolysiert;
(b) aus dem in der Verfahrensstufe (a) erhaltenen Reaktionsgemisch Ameisensäuremethylester und Methanol unter Bildung eines Ameisensäure und Wasser enthaltenden Stroms destillativ abtrennt;
(c) den Ameisensäuremethylester und gegebenenfalls Methanol enthaltenden Strom aus der Verfahrensstufe (b) durch
(i) Umsetzung mit einer basischen Verbindung mit einem pKₐ-Wert der korrespondierenden Säure der entsprechenden Dissoziationsstufe von ≥ 3, gemessen bei 25°C in wässriger Lösung, in Gegenwart von Wasser, und
(ii) destillativer Abtrennung des Methanols in einen Formiat und Wasser enthaltenden Strom überführt; und
(d) den Ameisensäure und Wasser enthaltenden Strom aus der Verfahrensstufe (b) und den Formiat und Wasser enthaltenden Strom aus der Verfahrensstufe (c) unter Bildung eines, das ameisensaure Formiat und Wasser enthaltenden Gemischs zusammenbringt.

2. Verfahren nach Anspruch 1, **dadurch gekennzeichnet, dass** man in der Verfahrensstufe (a) den Ameisensäuremethylester und das Wasser in einem Molverhältnis von 0,1 bis 1 zuführt.

3. Verfahren nach den Ansprüchen 1 bis 2, **dadurch gekennzeichnet, dass** man in der Verfahrensstufe (c) die destillative Abtrennung des Methanols und die Umsetzung des Ameisensäuremethylesters mit dem Wasser und der basischen Verbindung unter Überführung in den Formiat und Wasser enthaltenden Strom zusammen in einer Kolonne durchführt.

4. Verfahren nach den Ansprüchen 1 bis 3, **dadurch gekennzeichnet, dass** man in der Verfahrensstufe (d)
(i) den, die Ameisensäure und das Wasser enthaltenden Strom aus der Verfahrensstufe (b) zusammen mit der aus Schritt (iv) zurückgeführten Mutterlauge in einer Kolonne oder einem Verdampfer unter destillativer Abtrennung von Wasser aufkonzentriert;
(ii) den aus Schritt (i) durch Aufkonzentration gewonnen, Ameisensäure, Wasser und Formiat enthaltenden Strom mit dem, das Formiat und Wasser enthaltenden Strom aus der Verfahrensstufe (c) unter Bildung eines, das ameisensaure Formiat und Wasser enthaltende Gemisch zusammenbringt;
(iii) festes ameisensaures Formiat aus dem aus Schritt (ii) erhaltenen ameisensauren Formiat und Wasser enthaltenden Gemisch durch Kristallisation abscheidet und dieses isoliert; und
(iv) die erhaltene Mutterlauge zu Schritt (i) zurückführt.

5. Verfahren nach den Ansprüchen 1 bis 3, **dadurch gekennzeichnet, dass** man in der Verfahrensstufe (d)
(i) den, die Ameisensäure und das Wasser enthaltenden Strom aus der Verfahrensstufe (b) und den, das Formiat und das Wasser enthaltenden Strom aus der Verfahrensstufe (c) zu einem, das ameisensaure Formiat und Wasser enthaltende Gemisch in einer Kolonne oder einem Verdampfer unter destillativer Abtrennung von Wasser zusammenbringt; und
(ii) festes ameisensaures Formiat aus dem aus Schritt (i) erhaltenen ameisensauren Formiat und Wasser enthaltenden Gemisch durch Sprühgranulation, Sprühtrocknung oder Schmelzkristallisation abscheidet und dieses isoliert.

6. Verfahren nach den Ansprüchen 1 bis 5, **dadurch gekennzeichnet, dass** man in Verfahrensschritt (c) als basische Verbindung Natriumhydroxid, Natriumhydrogencarbonat, Natriumcarbonat, Kaliumhydroxid, Kaliumhydrogencarbonat, Kaliumcarbonat und/oder Ammoniak einsetzt.

7. Verfahren nach den Ansprüchen 1 bis 6, **dadurch gekennzeichnet, dass** man als ameisensaures Formiat ameisensaures Kaliumformiat, ameisensaures Natriumformiat, ameisensaures Kalziumformiat oder deren Gemische herstellt.

8. Verfahren nach den Ansprüchen 1 bis 7, **dadurch gekennzeichnet, dass** man als ameisensaures Formiat Kaliumdiformiat, Natriumdiformiat, Natriumtetraformiat oder deren Gemische herstellt.

9. Vorrichtung zur Herstellung von ameisensauren Formiaten gemäß der Ansprüche 1 bis 8, umfassend:
(a) einen, zur Hydrolyse von Ameisensäuremethylester geeigneten Reaktor (A);
(b) eine, zur destillativen Trennung eines Ameisensäuremethylester, Ameisensäure, Methanol und Wasser enthaltenden Stroms in Ameisensäuremethylester, Methanol und einen Ameisensäure und Wasser enthaltenden Strom geeignete Kolonne (B), welche zulaufseitig mit dem Reaktor (A) verbunden ist;
(c) eine, zur Verseifung von Ameisensäuremethylester mit einer basischen Verbindung und zur destillativen Abtrennung von Methanol geeigneten Kolonne (C), welche zulaufseitig mit dem Kolonnenkopf der Kolonne (B) verbunden ist und oberhalb des genannten Zulaufs eine Zulaufstelle für die basische Verbindung aufweist; und
(d) eine, zur Abtrennung von Wasser aus einem Ameisensäure und Wasser enthaltenden Strom geeignete Kolonne (D), welche zulaufseitig mit dem Kolonnensumpf der Kolonne (B) verbunden ist.

10. Vorrichtung nach Anspruch 9, umfassend
(e) einen, zur Kristallisation von ameisensauren Formiat geeigneten Apparat (E), welcher zulaufseitig mit dem Kolonnensumpf der Kolonne (D) und mit dem Kolonnensumpf der Kolonne (C) verbunden ist;
(f) einen, zur Abtrennung von Kristallen des ameisensauren Formiats geeigneten Apparat (F), welcher zulaufseitig mit Apparat (E) verbunden ist; und
(g) eine, zur Rückführung von Mutterlauge geeignete Verbindungsleitung (11) zwischen Apparat (F) und Kolonne (D).

11. Vorrichtung nach Anspruch 9, umfassend
(e) eine, zur Zuführung von wässrigem Formiat geeignete Verbindungsleitung (8) zwischen dem Kolonnensumpf der Kolonne (C) und der Kolonne (D); und
(f) einen, zur Sprühgranulation, Sprühtrocknung oder Schmelzkristallisation geeigneten Apparat (E), welcher zulaufseitig mit derm Kolonnensumpf der Kolonne (D) verbunden ist.

## Claims

1. A process for preparing acid formates which comprises
(a) partially hydrolyzing methyl formates with water;
(b) separating off by distillation methyl formate and methanol from the reaction mixture Obtained in process stage (a), forming a stream comprising formic acid and water;
(c) converting the stream comprising methyl formate with or without methanol from the process stage (b) by
(i) reaction with a basic compound having a pKₐ of the conjugate acid of the appropriate dissociation state of ≥3, measured at 25°C in aqueous solution, in the presence of water, and
(ii) removal of the methanol by distillation, into a stream comprising formate and water; and
(d) combining the stream comprising formic acid and water from the process stage (b) and the stream comprising formate and water from the process stage (c), forming a mixture comprising the acid formate and water.

2. The process according to claim 1, wherein, in the process stage (a), the methyl formate and the water are fed in a molar ratio of 0.1 to 1.

3. The process according to claims 1 to 2, wherein, in the process stage (c), the removal of the methanol by distillation and the reaction of the methyl formate with the water and basic compound with transfer into the stream comprising formate and water are carried out together in one column.

4. The process according to claims 1 to 3, wherein, in the process stage (d)
(i) the stream comprising the formic acid and the water from the process stage (b), together with the mother liquor recirculated from step (iv), is concentrated in a column or an evaporator with removal of water by distillation;
(ii) the stream which was produced from step (i) by concentration and comprises formic acid, water and formate is combined with the stream comprising the formate and water from the process stage (c) forming a mixture comprising the acid formate and water;
(iii) solid acid formate from the mixture comprising acid formate and water obtained from step (ii) is precipitated by crystallization and this is isolated; and
(iv) the resultant mother liquor is recirculated to step (i).

5. The process according to claims 1 to 3, wherein, in process stage (d)
(i) the stream from the process stage (b) comprising the formic acid and the water and the stream from the process stage (c) comprising the formate and the water are combined to form a mixture comprising the acid formate and water in a column or an evaporator with removal of water by distillation; and
(ii) solid acid formate is separated off by spray granulation, spray drying or melt crystallization from the mixture obtained from step (i) comprising acid formate and water, and this solid acid formate is isolated.

6. The process according to claims 1 to 5, wherein, in process step (c), the basic compound is sodium hydroxide, sodium hydrogen carbonate, sodium carbonate, potassium hydroxide, potassium hydrogen carbonate, potassium carbonate and/or ammonia.

7. The process according to claims 1 to 6, wherein the acid formate prepared is acid potassium formate, acid sodium formate, acid calcium formate or mixtures thereof.

8. The process according to claims 1 to 7, wherein the acid formate prepared is potassium diformate, sodium diformate, sodium tetraformate or mixtures thereof.

9. An apparatus for preparing acid formates according to claims 1 to 8, comprising:
(a) a reactor (A) suitable for hydrolyzing methyl formate;
(b) a column (B) suitable for separating by distillation a stream comprising methyl formate, formic acid, methanol and water into methyl formate, methanol and a stream comprising formic acid and water, which column is connected on the feed side to the reactor (A);
(c) a column (C) suitable for saponifying methyl formate with a basic compound and for removing methanol by distillation, which column is connected on the feed side to the column top of column (B) and has above said feed an inlet point for the basic compound; and
(d) a column (D) suitable for removing water from a stream comprising formic acid and water, which column is connected on the feed side to the column bottom of column (B).

10. The apparatus according to claim 9, comprising
(e) an apparatus (E) suitable for crystallizing acid formate, which apparatus is connected on the feed side to the column bottom of column (D) and to the column bottom of column (C);
(f) an apparatus (F) suitable for separating off crystals of the acid formate, which apparatus is connected on the feed side to apparatus (E); and
(g) a connection line (11) between apparatus (F) and column (D), which connection line is suitable for recirculating mother liquor.

11. The apparatus according to claim 9, comprising
(e) a connection line (8) between the column bottom of column (C) and column (D), which connection line is suitable for feeding aqueous formate; and
(f) an apparatus (E) suitable for spray granulation, spray drying or melt crystallization, which apparatus is connected on the feed side to the column bottom of column (D).

## Revendications

1. Procédé de préparation de formiates d'acide formique, **caractérisé par** les étapes consistant à :
(a) hydrolyser partiellement du méthylester d'acide formique avec de l'eau ;
(b) isoler du méthylester d'acide formique et du méthanol par distillation à partir du mélange réactionnel obtenu dans l'étape de procédé (a), avec formation d'un flux contenant de l'acide formique et de l'eau ;
(c) convertir le flux issu de l'étape de procédé (b), contenant du méthylester d'acide formique et éventuellement du méthanol, en un flux contenant du formiate et de l'eau par:
(i) réaction avec un composé basique présentant une valeur pKₐ de l'acide correspondant de l'étape de dissociation adéquate ≥ 3, mesurée à 25°C dans une solution aqueuse, en présence d'eau, et
(ii) isolation du méthanol par distillation dans un flux contenant du formiate et de l'eau ; et
(d) rassembler le flux issu de l'étape de procédé (b), contenant de l'acide formique et de l'eau, et le flux issu de l'étape de procédé (c), contenant du formiate et de l'eau, avec formation d'un mélange contenant le formiate d'acide formique et de l'eau.

2. Procédé selon la revendication 1, **caractérisé en ce que** le méthylester d'acide formique et l'eau sont amenés dans l'étape de procédé (a) en un rapport molaire de 0,1 à 1.

3. Procédé selon les revendications 1 à 2, **caractérisé en ce que** l'isolation du méthanol par distillation et la réaction du méthylester d'acide formique avec l'eau et le composé basique avec conversion en un flux contenant du formiate et de l'eau sont mises en oeuvre ensemble dans une colonne, dans l'étape de procédé (c).

4. Procédé selon les revendications 1 à 3, **caractérisé par** les phases consistant, dans l'étape de procédé (d), à :
(i) reconcentrer le flux issu de l'étape de procédé (b), contenant l'acide formique et l'eau, en commun avec l'eau-mère remise en circulation à partir de la phase (iv), dans une colonne ou un évaporateur avec isolation de l'eau par distillation ;
(ii) rassembler le flux contenant de l'acide formique, de l'eau et du formiate, obtenu par reconcentration à partir de la phase (i), avec le flux issu de l'étape de procédé (c), contenant du formiate et de l'eau, avec formation d'un mélange contenant le formiate d'acide formique et de l'eau ;
(iii) précipiter du formiate d'acide formique solide par cristallisation à partir du mélange contenant du formiate d'acide formique et de l'eau, obtenu à la phase (ii), et isoler celui-ci ; et
(iv)remettre en circulation dans la phase (i) l'eau-mère obtenue.

5. Procédé selon les revendications 1 à 3, **caractérisé par** les phases consistant, dans l'étape de procédé (d), à :
(i) rassembler dans une colonne ou un évaporateur le flux issu de l'étape de procédé (b), contenant l'acide formique et l'eau, et le flux issu de l'étape de procédé (c), contenant le formiate et l'eau, pour donner un mélange contenant le formiate d'acide formique et de l'eau, avec isolation de l'eau par distillation ; et
(ii) séparer du formiate d'acide formique solide par granulation par pulvérisation, séchage par atomisation ou cristallisation en fusion à partir du mélange contenant du formiate d'acide formique et de l'eau obtenu à la phase (i), et isoler celui-ci.

6. Procédé selon les revendications 1 à 5, **caractérisé en ce que** de l'hydroxyde de sodium, de l'hydrogénocarbonate de sodium, du carbonate de sodium, de l'hydroxyde de potassium, de l'hydrogénocarbonate de potassium, du carbonate de potassium et/ou de l'ammoniac sont utilisés dans l'étape de procédé (c) en tant que composé basique.

7. Procédé selon les revendications 1 à 6, **caractérisé en ce que** du formiate de potassium d'acide formique, du formiate de sodium d'acide formique, du formiate de calcium d'acide formique ou leurs mélanges sont préparés en tant que formiate d'acide formique.

8. Procédé selon les revendications 1 à 7, **caractérisé en ce que** du diformiate de potassium, du diformiate de sodium, du tétraformiate de sodium ou leurs mélanges sont préparés en tant que formiate d'acide formique.

9. Dispositif de préparation de formiates d'acide formique selon les revendications 1 à 8, comportant :
(a) un réacteur (A) approprié pour l'hydrolyse de méthylester d'acide formique ;
(b) une colonne (B) appropriée pour la séparation par distillation d'un flux contenant du méthylester d'acide formique, de l'acide formique, du méthanol et de l'eau en méthylester d'acide formique, en méthanol et en un flux contenant de l'acide formique et de l'eau, qui est reliée en amont avec le réacteur (A) ;
(c) une colonne (C) appropriée pour la saponification de méthylester d'acide formique avec un composé basique et pour l'isolement par distillation de méthanol, qui est reliée en amont avec la tête de colonne de la colonne (B) et présente au-dessus de l'arrivée mentionnée un point d'arrivée pour le composé basique ; et
(d) une colonne (D) appropriée pour l'isolation d'eau à partir d'un flux contenant de l'acide formique et de l'eau, qui est reliée en amont avec le pied de colonne de la colonne (B).

10. Dispositif selon la revendication 9, comportant:
(e) un appareil (E) approprié pour la cristallisation de formiate d'acide formique, qui est relié en amont avec le pied de colonne de la colonne (D) et avec le pied de colonne de la colonne (C) ;
(f) un appareil (F) approprié pour l'isolation de cristaux du formiate d'acide formique, qui est relié en amont avec l'appareil (E) ; et
(g) une canalisation de jonction (11) entre l'appareil (F) et la colonne (D), appropriée pour la remise en circulation de l'eau-mère.

11. Dispositif selon la revendication 9, comportant:
(e) une canalisation de jonction (8) entre le pied de colonne de la colonne (C) et la colonne (D), appropriée pour l'acheminement de formiate aqueux ; et
(f) un appareil (E) approprié pour la granulation par pulvérisation, le séchage par atomisation ou la cristallisation en fusion, qui est relié en amont avec le pied de colonne de la colonne (D).
